# EUROPEAN PATENT SPECIFICATION

(11) **EP 3 203 923 B1**
(45) Date of publication and mention of the grant of the patent: **01.04.2020**
(21) Application number: 15848431.1
(22) Date of filing: 27.07.2015
(51) Int. Cl.: A61B 50/30, A61B 50/33, A61B 50/20, A61B 50/00

(54) **ORGANIZER FOR SURGICAL INSTRUMENTS AND ITEMS USED DURING SURGERY**
ORGANISATOR FÜR CHIRURGISCHE, WÄHREND EINER OPERATION VERWENDETER INSTRUMENTE UND ARTIKEL
DISPOSITIF DE RANGEMENT POUR INSTRUMENTS CHIRURGICAUX ET ARTICLES UTILISÉS PENDANT UNE CHIRURGIE

(30) Priority: 08.10.2014 US 201414509986; 13.04.2015 US 201514685465; 13.07.2015 US 201514798327
(43) Date of publication of application: 16.08.2017
(73) Proprietor: Richman, Lawrence, Los Angeles, California 90048 (US)
(72) Inventor: Richman, Lawrence, Los Angeles, California 90048 (US)
(74) Representative: Patentanwälte Olbricht Buchhold Keulertz
(86) International application number: PCT/US2015/042307
(87) International publication number: WO 2016/057102

(56) References cited:
- DE-U1- 20 318 732
- US-A- 3 987 895
- US-A- 4 011 944
- US-A- 4 153 160
- US-A1- 2006 175 209
- US-A1- 2009 266 728
- US-B1- 6 426 041
- US-B2- 7 066 328
- US-B2- 7 997 847
- US-B2- 8 177 064
- Anonymous: "Developmentally Friendly - Neonatal and Pediatric Intensive Care", UTAH MEDICAL PRODUCTS, INC, 30 June 2013 (2013-06-30), XP055426555, Retrieved from the Internet: URL:http://www.utahmed.com/pdf/58105.pdf [retrieved on 2017-11-20]
- UTAH MEDICAL PRODUCTS, INC.: 'Developmentally Friendly - Neonatal and Pediatric Intensive Care' 30 June 2013, XP055426555 Retrieved from the Internet: <URL:http://www.utahmed.com/pdf/58105.pdf>
- UTAH MEDICAL PRODUCTS, INC.: 'Umbili-Cath' 30 June 2013, XP009501733 Retrieved from the Internet: <URL:https://web.archive.org/web/2013063006 1754/ http://www.utahmed.com/umbilicath.htm> [retrieved on 2015-10-05]

## Description

### BACKGROUND

1. *Field:* Organizers for holding surgical instruments in surgical suites.
2. *Related Applications:* This patent claims priority from the following utility patent applications: Application No. 14/509,986, filed October 8, 2014, "Organizer for Surgical Tools and Items Used during Surgery." Application No. 14/685,465, filed April 13, 2015, "Organizer for Surgical Instruments and Items Used during Surgery." Application No. 14/798,327, filed July 13, 2015, "Organizer for Surgical Instruments and Items Used during Surgery." Applicant claims priority based upon those applications.
3. *General Background and State of the Art:* Surgeons and their staff need their instrument and other items used during surgery to be readily accessible and well organized. Spending time looking for missing instruments hide adds crucial time to a surgery. That is undesirable for the patient because delays increase the length of the surgery and time under anesthesia.

Increasing the time for each surgery also is detrimental to surgeons, accompanying physicians and staff and to the hospital or surgical center. Surgeons and other physicians and staff are in high demand and often perform many procedures daily. Adding time to each surgery can eliminate one or more surgeries per day or force a surgery planned for one day to be delayed to the next or a later day.

For hospitals and surgical center, delays for each surgical team compound. The surgical suites at many hospitals are fully utilized. That fact alone causes delays in scheduling patients' surgeries. In addition, if some or all the surgical suites handle even one fewer procedures every day, the hospital becomes less efficient. Therefore, costs increase. Because fully equipped surgical suites are very expensive, adding more surgical suites is costly. On the other hand, having any added but under-utilized surgical suites generates less revenue to pay for the surgical suites or for other hospital expenses.

Increased costs and decreased efficiency are not the only concerns. At the end of each procedure, the surgical team must account for all instruments. For example, if the surgeon starts with 15 surgical clamps and has five unused surgical clamps left at the end of the surgery, the other ten must be accounted for. Otherwise, a missing clamp might be inside the patient. More likely, it may be hidden on the operating table or dropped on the floor. Finding the clamps may not be difficult and may not take excessive time, but the time spent adds up for each surgical suite, each hospital and hospitals in general.

A document "Developmentally Friendly - Neonatal and Pediatric Intensive Care", UTAH MEDICAL PRODUCTS, INC, June 30, 2013, XP055426555 and the patent US 8 177 064 B2 describe a tray with instruments placed in wells having a shape corresponding to the outer shape of each instrument.

### SUMMARY

The invention provides an organizer for holding surgical instruments according to claim 1. Further embodiments of the invention are provided in the dependent claims. An organizer for holding surgical instruments includes a tray that has indentations extending down from the top surface of the tray. The indentations form instrument wells. Each instrument well has a shape that corresponds to the shape of particular surgical instruments. For example, the instrument well for a scissors has two round regions to receive the scissors' finger openings, a rectangular region extending from the round regions and a narrow region corresponding to the scissors' pointed end. Likewise, the instrument well for scalpels has a tapered length corresponding to the handle and a narrower region for the blade.

The depth of each instrument well corresponds to the height of a stack of the surgical instruments that a surgeon plans to use. For example, if a particular surgery typically uses three scalpels, the depth of the scalpel instrument well would accommodate the three scalpels. If another type of surgery uses more than three scalpels, the instrument well would be deeper. Otherwise, more than one instrument well could be used with the scalpels divided between the wells.

The tops of the stack may be aligned with the tray's top surface or the stacks should be the same, short distance below that surface. After surgery, the used instruments are returned to their instrument well. Thus, at a glance, one can tell whether the instrument well is full. If any instrument wells are not full, it indicates an instrument is missing, which alerts surgical suite personnel that they must find the missing instrument.

Alternatively, a complimentary set of instrument wells may be provided. Each instrument well in the complimentary set is the same or almost the same shape and depth as one of the first-mentioned instrument well. After a surgical instrument is used, it is put into its proper instrument well in the complimentary set instead of returning it to the first-mentioned instrument well

The tray has locking bars at the top or upper surface of the tray, which extend over each instrument well. In the locked position, instruments cannot be added to or removed from the instrument well. The bar can pivot or otherwise move to a position uncovering the instrument well so that instruments can be the removed or added to the instrument well. When the bar extends over the instrument well, the bar will be against the top instrument in the instrument well if the well is full. Seeing that contact between the bar and the top instrument allows one to see quickly whether the instrument well is full. If a surgical instrument is missing, the far end of the locking bar may be offset to project upwards to alert the operating staff of the missing instrument.

If the instruments are returned to their instrument well or to the initially empty well following surgery, each instrument well that contains the used instruments should contain the same number of instruments that filled the instrument well when the surgery started. If one or more instruments are missing from their instrument well, the locking bar's intersection with the structure of the tray adjacent the instrument well is such that an end of the locking bar projects about the tray's surface. Thus, one can notice quickly whether all instrument wells are full because all surgical instruments are returned to their indentation.

Plastic, a potential material for the tray, is not a good conductor of heat. If the surgical instruments in the instrument wells are to be sterilized by high temperature and pressurized steam in an autoclave, the plastic of the instrument wells may prevent complete sterilization of the surgical instruments. If the instrument wells are not solid, the high-temperature steam can reach the surgical instruments so that the instruments can be sterilized. To make the walls of the wells not solid, they can be formed of spaced plastic strips or arms. Perforating the walls of the wells with sufficient open space also would allow the steam to reach the instruments.

To assist the surgical suite staff further, part or the entire top of the locking bar may be colored green or another color so that when the locking bar in the closed position over the instruments, the surgeon or staff member can see that the bar is closed. Similarly, part or all of the opposite side of the locking bar may be colored red or another color different from the first side of the locking bar. Therefore, one will see red when the locking bare is in the open position when instruments are being used. At the end of the operation, the surgical suite staff can be assured that all instruments are accounted for when all the locking bars are fully flush with the tray and show green over each instrument well. Instead of color, the top and bottom of the locking bar may have contrasting symbols.

Alternatively, the organizer may contain a locking plate that pivots about an axis perpendicular to the tray's top surface (this alternative do not fall under the claimed scope of protection). In an unlocked orientation, the locking plate is out of the way of the surgical instruments in the instrument well. Pivoting the locking plate about its axis moves part of the locking plate over the top-most surgical instrument to lock the instruments in the instrument well. The locking plate may pivot into a small cavity at the top surface of the tray. Pivoting the locking plate into the cavity unlocks the-instruments in the instrument and covers the top surface of the cavity. Coloring the cavity's top surface indicates whether the locking plate is locked. That is, if the color of the cavity is visible, the locking plate is locked. If the user does not see color, the locking plate is unlocked. Thus, after all instruments are returned to their proper instrument well and all the locking plates are locked, color should be visible for every lock.

A spring can mount below the bottom-most instrument to urge the instruments upward toward or above the tray's top surface. A plunger may be mounted adjacent the instrument well (this arrangement do not fall under the claimed scope of protection). A base extending from an upright portion of the plunger extends under the bottom-most instrument in the instrument well, and the spring mounts below the base of the plunger. Then at least one instrument is removed from the well, the spring
urges the plunger upward such that the top of the upright portion of the plunger extends above the top of the tray. After surgery and after all the instrument are returned to their respective instrument wells, one can determine if any instruments are missing because the top of at least one plunger extends about the tray's top surface.

### BRIEF DESCRIPTION OF THE DRAWINGS

Fig. 1 is a perspective view one version of the tray showing instruments in the instrument wells.
Fig. 2 is a plan view one version of the tray showing instruments in the instrument wells.
Fig. 3 is a side, sectional view one version of the tray taken through plane 3-3 of Fig. 2.
Figs. 4 and 5 are sectional views of one instrument well at the tray's top surface.
Figs. 6, 7, 8 and 9 are perspective views of one instrument well at the tray's top surface showing the locking bar in different orientations.
(The description regarding the second version of the tray do not form subject-matter falling under the scope of protection. This relates to the Fig. 10 to Fig. 20 and Fig. 25 to Fig 27) Fig. 10 is a perspective view a second version of the tray showing instruments in the instrument wells.
Fig. 11 is a perspective view the second version of the tray showing part of the tray folded relative to another part of the tray.
Fig. 12 is a perspective view the second version of the tray showing part of the tray fully folded relative to another part of the tray.
Fig. 13 is a plan view the second version of the tray in its "open" configuration. The drawing shows instruments in the instrument wells.
Fig. 14 is a side, sectional view the second version of the tray taken through plane 14-14 of Fig. 13.
Figs. 15, 16 and 17 are sectional views of a portion of an instrument well showing detail 15 in Fig. 14.
Fig. 18 is a sectional view of a portion of an instrument well taken through plane 18-18 in Fig. 13.
Figs. 19 and 20 are perspective views of one instrument well at the tray's top surface showing a locking bar in different orientations.
Fig. 21 is a perspective view of a modified organizer in its closed position.
Figs. 22 and 23 are perspective views of a modified tray.
Fig. 24 is a perspective view of another modified tray.
Fig. 25 is a perspective view, which is partially cutaway, showing the detail of a modified instrument well.
Figs. 26 and 27 are sectional views taken through planes 26-26 and 27-27 of Fig. 25.
Fig. 28 is a partially cutaway, perspective view, showing a modified instrument well.
Fig. 29 is sectional view taken through plane 29-29 in Fig. 28.

### DETAILED DESCRIPTION

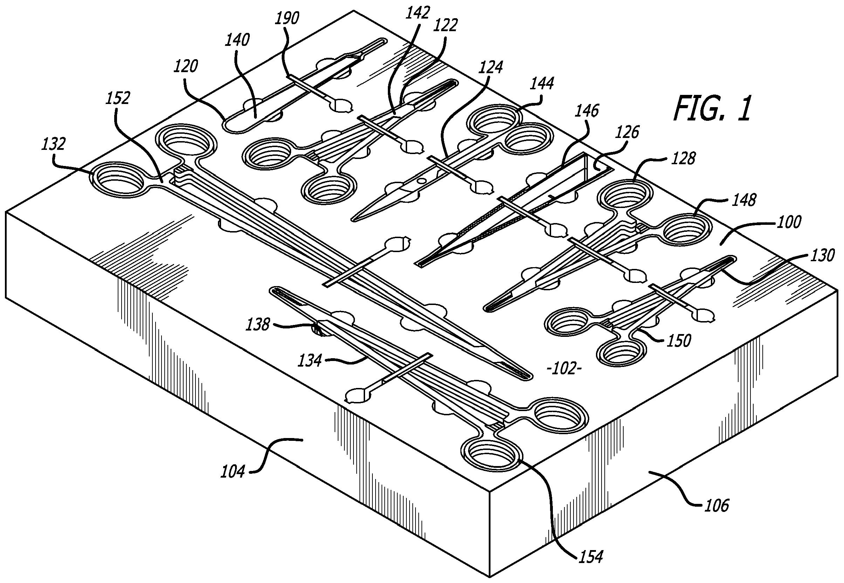

Tray 100 (Figs. 1, 2 and 3) for holding surgical instruments may be made from any suitable material such as plastic or non-corrosive metal such as stainless steel, but cardboard, formed paper and composites are options. The non-metal materials may be easier to form and are less costly than metal. The material for the tray shown in the drawings is rigid, but the material could be bendable.

Tray 100 may be disposable. However, non-disposable materials should be able to retain their form when subjected to autoclave temperatures (100°C at 137.9 kPa (20 psi)) or whatever temperatures and pressures are customary for a particular facility.

Tray 100 shown in the drawings has a top surface 102 and depending sidewalls, only three of the four, 104, 106 and 108, are visible in the drawings. The tray is rectangular, but other shapes such as polygons, circles, ellipses and other freeform shapes could be acceptable.

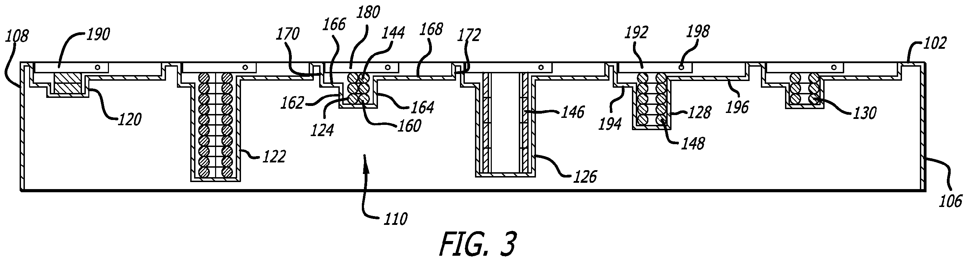

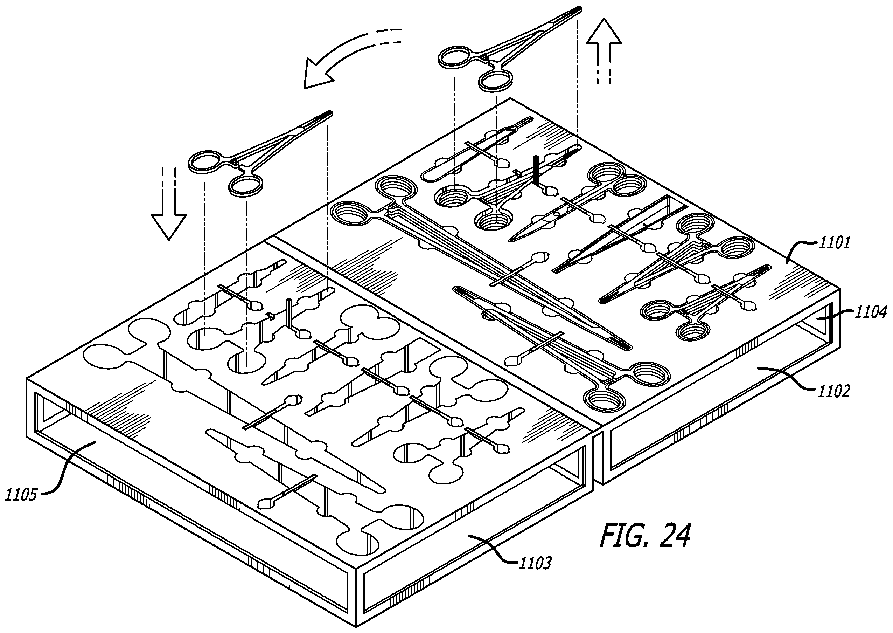

The base of tray 100 is open, but it could be closed. With the base open, one has access to the tray's underside 110 (Fig. 3). See also Fig. 24, which is discussed below, where the sides of the tray are open for high-pressure and temperature steam in an autoclave to reach the surgical instruments.

Top surface 102 of tray 100 has several indentations that form instrument wells for receiving surgical instruments. Eight instrument wells, 120, 122, 124, 126, 128, 130, 132 and 134, are shown in the drawings, but the tray could have more or fewer instrument wells. Rather than adding many instrument wells to make a tray that may be too large, two or more trays of a desired size could replace a single, large tray.

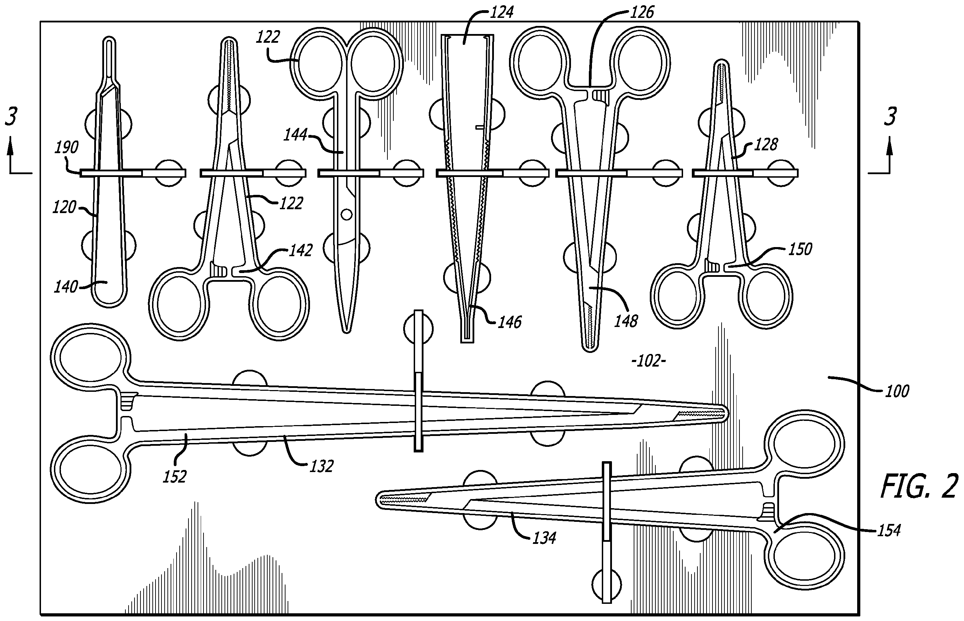

Each instrument well is shaped to receive a particular instrument. For example, well 120 conforms to the shape of scalpels 140 (Figs. 1 and 2). Instrument well 122 receives surgical clamps142. Scissors 144 fit into instrument well 144, and forceps 146 fit into instrument well 126. Well 128 holds more clamps148. Wells 130, 134 and 132 receive small, medium and large needle holders 150, 154 and 152, respectively. The instrument wells may have depressions 138 at appropriate places to allow easier gripping of an instrument.

Fig. 3 shows that the instrument well may have different depths to accommodate different sizes and numbers of surgical instruments. For example, consider instrument well 124 in Fig. 3, which receives scissors 144. The indentation that forms the instrument well includes base 160, which depends from sidewalls 162 and 164. The tops of the sidewalls intersect shoulders 166 and 168, and the outside of each shoulder intersects short extension 170 and 172. The extensions intersect top surface 102 of tray 100. The functions for the space above the shoulders are discussed below.

Experienced surgeons anticipate using a specific number of each surgical instrument for a specific surgery. Accordingly, the number of instrument wells and their shapes for particular instruments could vary for specific surgeries.

Consider a surgeon who anticipates for a particular procedure needing three scissors of the size of scissors 144 in Fig. 1. Thus, for that surgery, the instrument well is deep enough to hold three scissors - no more and no fewer. Similarly, if the surgeon anticipates needing four forceps, instrument well 126 is deep enough to hold four forceps 146. Because the forceps are thicker than the thickness of scissors 144, the instrument well 126 holding four forceps is much deeper than the instrument well 124 holding three scissors. The deepest instrument well, 122, holds ten surgical clamps 142.

A leaf or other spring (not shown) could be used at the bottom of some or all the instrument wells to urge the instruments upward. Such an arrangement could assist in gripping the top-most instrument.

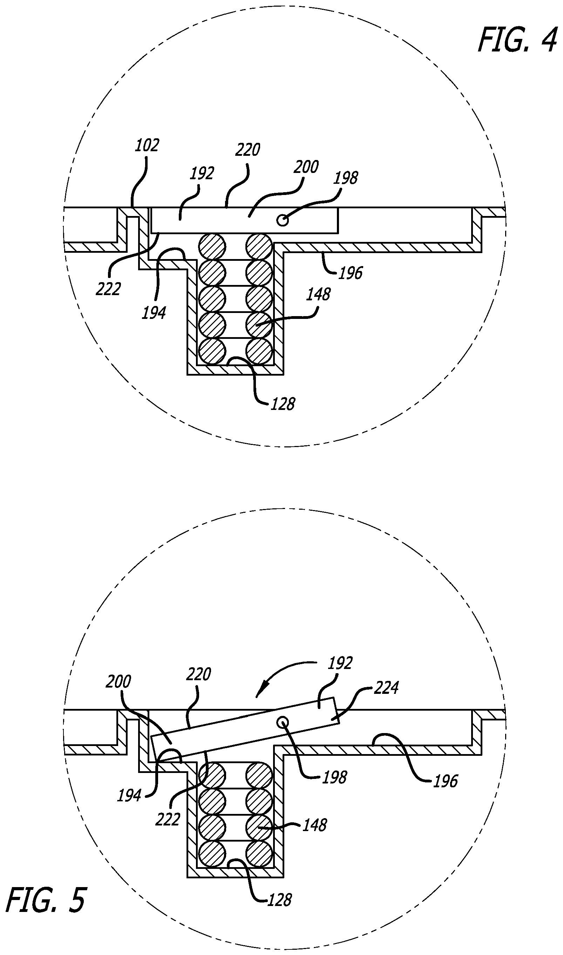

A locking bar extends over the top-most instrument in each instrument well. The locking bars are similar; only locking bars 190 and 192 are discussed. Instrument well 128 holds five forceps or surgical clamps 148 (Fig. 4). Locking bar 192 mounts on pin 198 in the space above shoulders 194 and 196. The locking bar's top face 200 is in the same plane as top surface 102 of tray 100. The locking bar is seen resting on the upper-most forceps in Fig. 4.

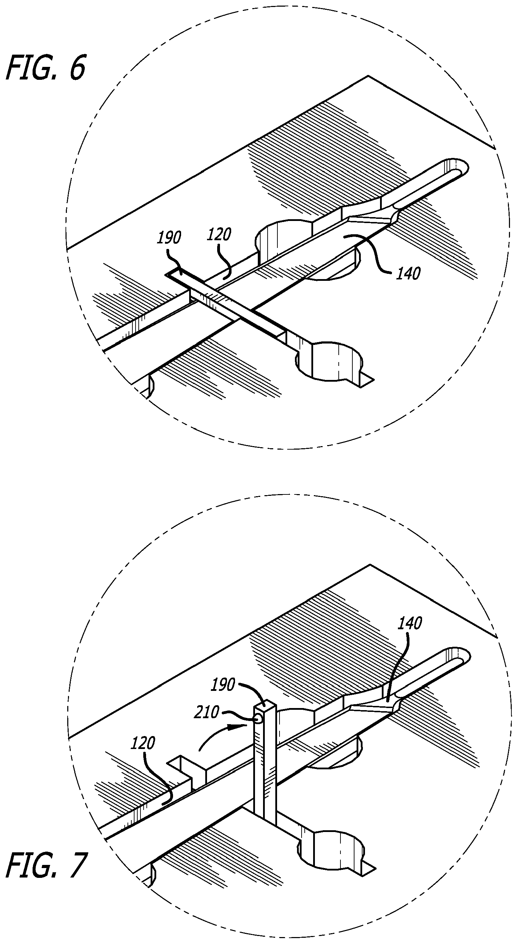

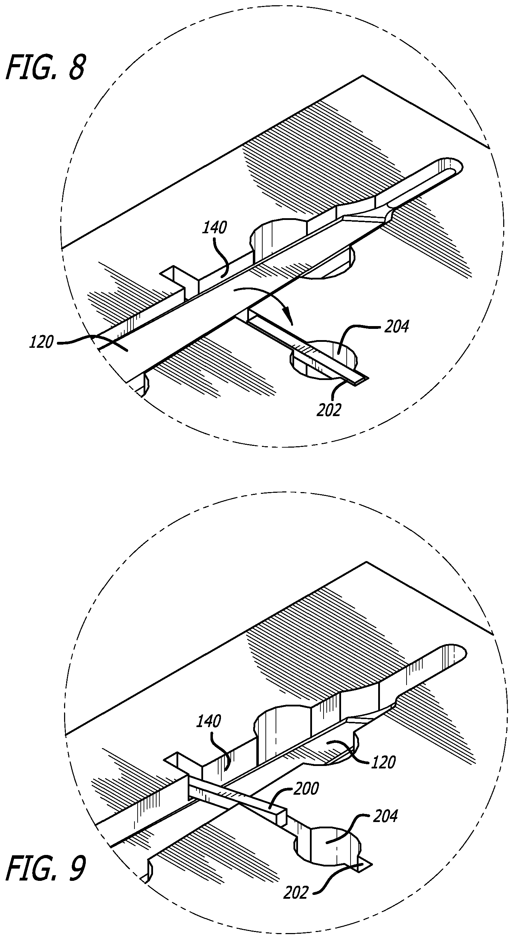

To remove an instrument from an instrument well, the locking bar is pivoted to or past vertical. Thus, as Figs. 6 and 7 show, locking bar 190 pivots about its pin (not shown) from the horizontal position over instrument well 120 and scalpels 140 (Fig. 6) to a vertical orientation (Fig. 7). The locking bar can continue to rotate to the Fig. 8 position where it rests in groove 202. In that position (and it the Fig. 6 position), a person moving his or her hand over the top 102 of tray 100 will encounter no obstructions from the locking bars. The groove also may have depressions 204 that allow insertion of a finger or instrument to pull the locking bar out of the groove. The locking bars may have a springloaded pin 210 that engages a detent (not shown) in the groove. That arrangement tends to hold the locking bar over the instruments until one rotates the locking bar away from the instruments.

The locking bar may have different colors on its top and bottom. All or part of top face 220 (Fig. 4) could be colored green, for example, which would show that all locking bars are in their locked or almost locked (Fig. 5) position. All or part of bottom face could be colored red, for example, so that the red face would show when the locking bar is open to allow access to instrument well 148. Symbols could replace the colors.

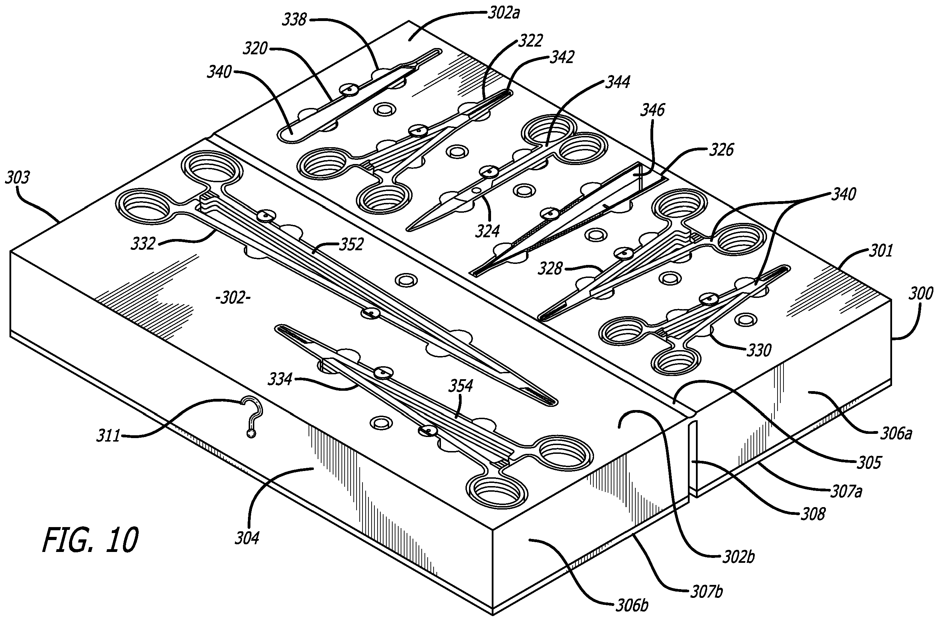

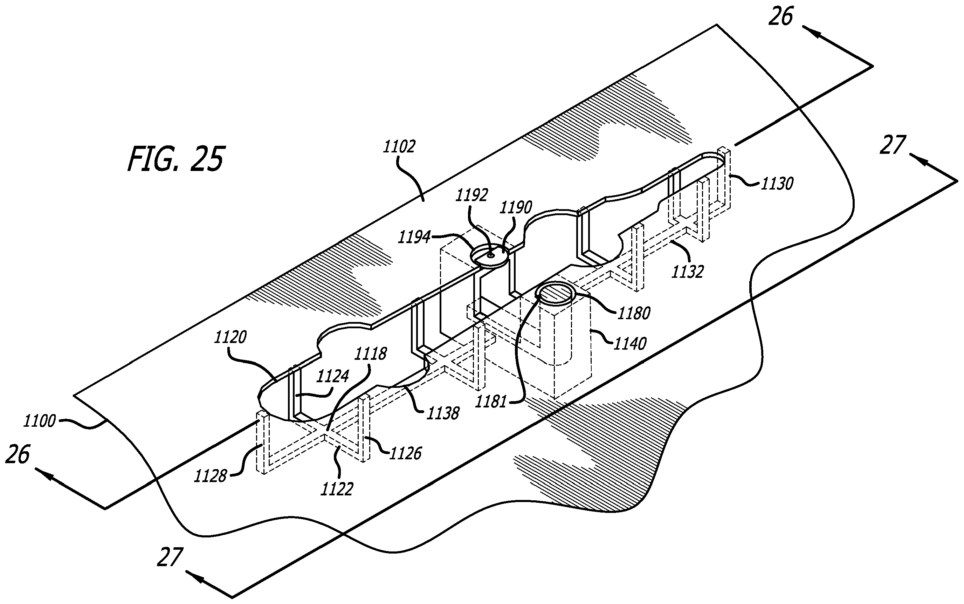

In the drawings, the locking bars pivot over and away from the instrument wells. Other arrangements are possible although they may not offer all the advantages of the pivoting locking bars. For example, the locking bar could slide in an elongated groove from a position over the instrument well to a position spaced from the instrument well. Likewise, a U-shaped fastener could be inserted into openings adjacent the sides of each instrument well. The tray shown in Figs. 10 and 25 uses a pivoting locking plate that in one position, blocks instruments' insertion and removal but in its other position, it does not block the instrument wells. Other arrangements also are possible.

When surgery begins, the surgeon or assistant opens all the locking bars, e.g., bars 190 and 200, of fully loaded tray 100. Of course, not all locking bars must be opened in the beginning, but doing so may be more convenient. As the surgery proceeds, the surgeon and his or her staff use the instruments as necessary until the surgery is finished. Then the instruments are returned to their original instrument well.

When all five forceps or surgical clamps 148 are returned to instrument well 128 and locking bar 200 is pivoted to its lock position, the locking bar aligns with the top surface 102 of tray 100. See Fig. 4. However, if only four clamps are returned to their instrument well, when the locking bar is pivoted to the lock position, the end of the locking bar pivots further until it contacts shoulder 194. See Fig. 5. Consequently, the right side of the locking bar (Fig. 5) projects above the top surface of the tray. That is because pin 198, which acts as a fulcrum is closer to the short side 224 of the locking bar. Thus, one knows at a glance or by running a hand over the tray that at least one forceps is missing from its instrument well.

Likewise, if fewer than two scalpels 140 are returned to instrument well 120, locking bar 200 will not be flat. See Fig. 9. One can easily tell that at least one scalpel is missing.

Thus, one advantage of having pivoting locking bars such as bars 190 or 200 is their orientation projecting above surface 102 of tray 100 when the instrument well is not full. Upon finding an instrument missing from the tray, those in the surgical suite can search for the instrument. Because the search is part of the time spent returning the instruments to the tray, locating all the instruments used is handled at one time and becomes more efficient.

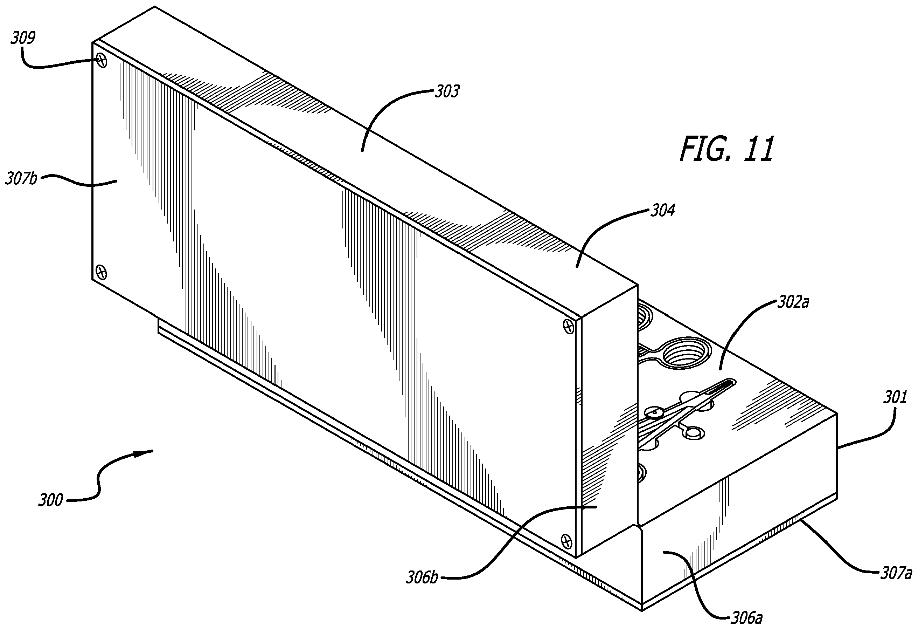

A second version of the organizer includes tray 300 (Figs. 10, 11 and 12). The material for the tray is similar to the materials of tray 100 (Fig. 1). The material for the tray shown in the drawings is rigid, but the material could be bendable.

Tray 300 may be divided in at least two sections 301 and 303. The two sections that Figs. 1 through 3 show may be hinged at 305 together to allow one section to fold over the other. Compare Fig. 1 with Figs. 2 and 3, in which Fig. 1 is open, Fig. 3 is closed and Fig. 2 is between open and closed. The tray may have structure for securing the two sections in the closed position. For example, hook 311 (Fig. 10) on section 303 can engage a pin, eye or other structure (not shown) on section 301.

If the tray is plastic, hinge 305 could be a plastic living hinge, which is formed during injection molding. Fabric, metal or plastic or other types of hinges could be used instead of a living hinge.

Tray 300 may be disposable. However, any non-disposable materials should be able to retain their form when subjected to autoclave temperatures.

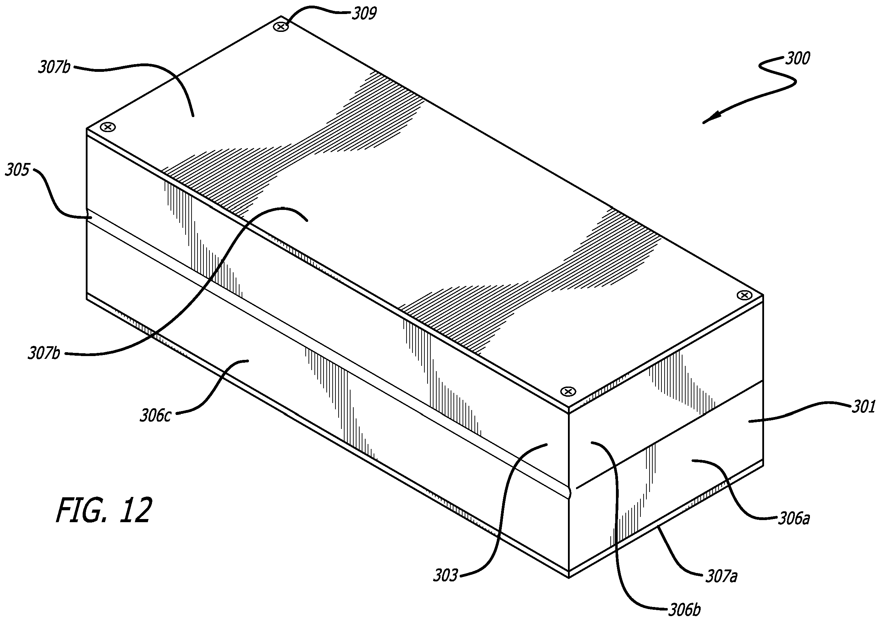

Tray 300 shown in the drawings includes a top surface 302 that is divided into surface regions 302a and 302b. The tray is rectangular, but other shapes could be acceptable. The tray also has depending sidewalls, only three of which, 304, 306a and 306b, are visible in Fig. 10. See also sidewall 306c (Fig. 12). The two sections 301 and 303 may be spaced apart along gap 308 when the tray is in its open configuration (Fig. 10).

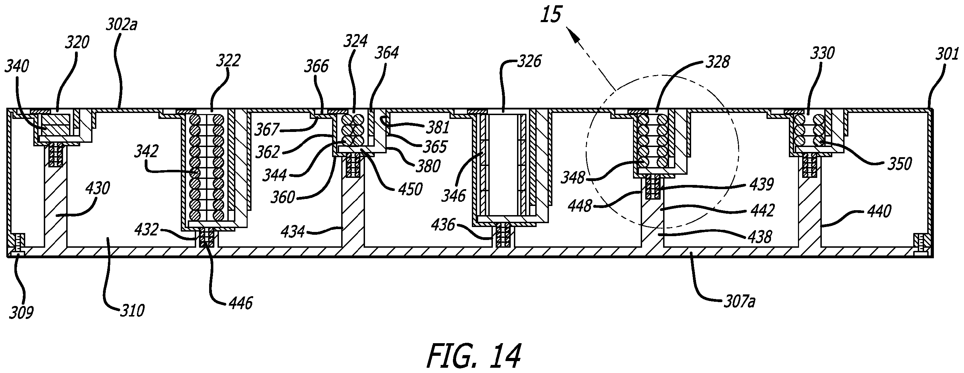

The base of tray 300 may be open, but the base is closed in this version. That is, plates 307a and 307b cover the base. (Figs. 10, 11, 12 and 14). The plates may be removable. Therefore, the plates are fastened with screws 309 or other fasteners to the base of the tray. Removing the plates allows access to the tray's inside 310 (Fig. 14).

Indentations in top surface 302 of tray 300 form instrument wells that receive surgical instruments. The drawings show eight instrument wells, 320, 322, 324, 326, 328, 330, 332 and 334, but the tray could have more or fewer instrument wells.

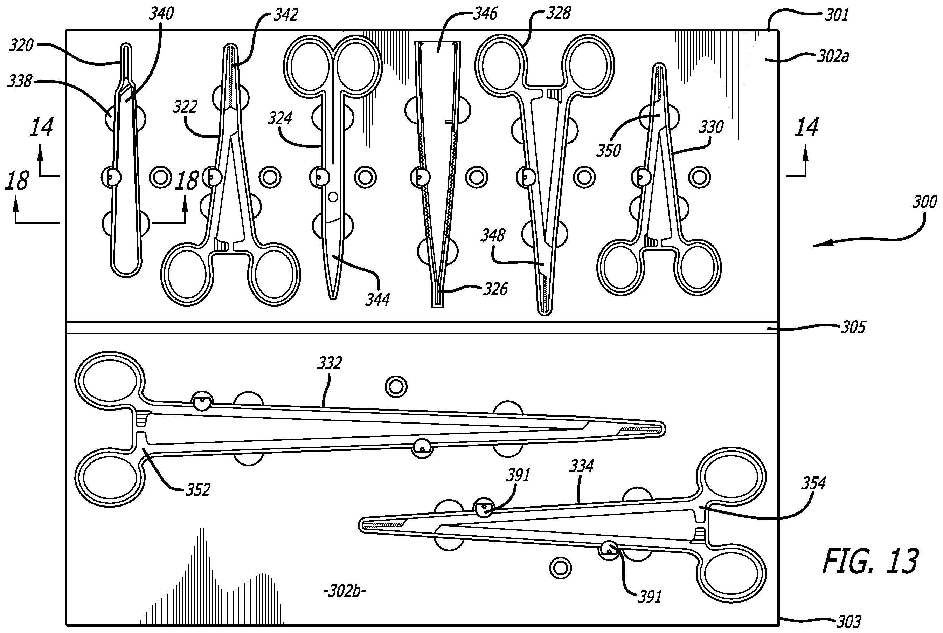

Positioning the surgical instruments on one side or the other of surface 302 facilitates opening and closing of the tray. See Figs. 10 and 13, which show no instrument extending past hinge 305. Note that the axis of each instrument wells is perpendicular to the hinge, but angling one or more of the instrument wells could accommodate longer instruments. Likewise, orienting the instrument wells differently may allow the tray to hold more instruments.

The shape of each instrument well conforms to that of a particular instrument. For example, the shape of well 320 conforms to the shape of scalpels 340 (Figs. 10 and 13). Instrument well 322 receives surgical clamps 342. Scissors 344 fit into instrument well 344, and forceps 346 fit into instrument well 326. Well 328 holds additional clamps 348. Instrument wells 330, 334 and 332 receive small, medium and large needle holders 350, 354 and 352, respectively. The instrument wells may have depressions such as depression 338 at appropriate places to allow easier gripping of an instrument by one's fingers or with an instrument. See Figs. 10, 13, 18, 19 and 20.

The instrument wells may have different depths to accommodate different numbers of surgical instruments and instruments of different dimensions. For example, consider instrument well 324 for scissors 344 in Fig. 14's sectional view. The indentation that forms the instrument well includes bottom 360, which is at the bottoms of sidewalls 362 and 364. The top of the sidewall 362 intersect shoulder 366, which extends into a short extension 367, which intersects top surface 302a. Sidewall 364 extends to top surface 302a.

Bases 307a and 307b have pillars extending upward from the base to support the respective instrument wells. The drawings only show the pillars for base 307a. See Fig. 14, which show pillars 430, 432, 434, 436, 438 and 440. Each pillar 430, 434, 438 and 440 has a pedestal section with a cavity on top. See cavity 439 on pedestal 442 in Figs. 14, 15, 16 and 17. Because instrument well 322 is deeper than other wells and extends almost to base 307a, pillar 432 needs no pedestal section to raise its cavity 433 high enough for its surgical instruments to reach near surface 302a. Thus, its cavity extends to base 307a. Because the pillars, pedestals and cavities perform similar functions, only the structure for pillars 432 and 438 are discuss further in detail.

Each cavity contains a coil spring. See spring 448 in Figs. 14, 15, 16 and 17 and spring 446 in Fig. 14. Leaf springs or other types of resilient structure could substitute for coil springs. In the drawings, the cavities may be cylindrical to receive round coil springs. If other types of springs are used, the cavities can be sized to contain the springs. If so, the pillars may be sized to accommodate different sizes and shapes for the cavities.

Springs such as springs 446 and 448 extend through the base of their respective instrument well, e.g., bottom 360 of well 324. The spring urges arm 450 of plunger 380 upward. Likewise, spring 448 extends through an opening in base 449, where it urges arm 456 of plunger 452 upward. The upward force from each spring urges the surgical instrument within the respective instrument well upward toward top surface 302a or 302b.

Plunger 380 mounts in opening 381. See Figs. 10, 13 and 14. Fig. 25 also shows a plunger, but it will be discussed in conjunction with the discussion of that figure. Corresponding plungers mount next to each of the other instrument wells. Plunger 380 (Fig. 14) includes upright portion 382 and base 384 at the bottom of the upright portion. The base of the plunger extends into instrument well 324 below the surgical instrument, scissor 344, which is the instrument in instrument well 324.

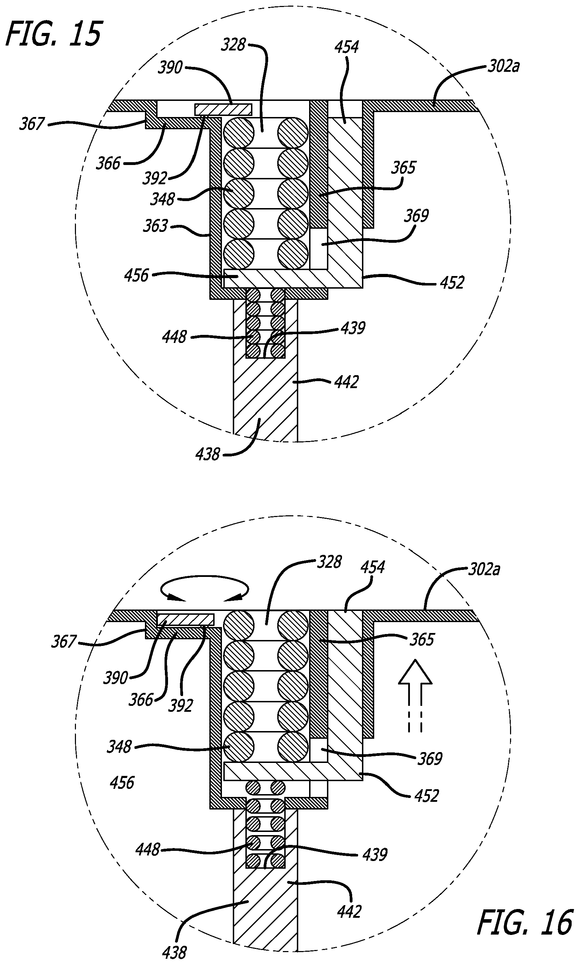

Figs. 15, 16 and 17 shows plunger 452 for instrument well 328 in more detail. The plunger includes upright portion 454 and base 452. The plunger's base extends below surgical clamps 348, which are in instrument well 324. When instrument well 328 is full, i.e., containing five surgical clamps, locking plate 390 hold the clamps in place against the upward force from spring 448. The locking plates are described below.

Assume that during a surgical procedure, a surgeon plans to use fifteen surgical clamps of the size of clamps 342 and 348 (Figs. 14, 15, 16 and 17). Ten clamps 342 are in well 322. Thus, for that surgery, the other instrument well, well 328, should be deep enough to hold five clamps-no more and no fewer. Though the version shown in Fig. 14 divides the clamps ten in one well 322 and five in the other well 328, the depth of each well could have a total depth to receive fifteen clamps divided nine and six, eight and six or some other division. Similarly, if the surgeon anticipates needing four forceps, instrument well 326 is deep enough to hold four forceps 346. Because the height of the forceps are greater than the clamps' height, instrument well 326, which holds four forceps, is deeper than the instrument well 328 holding three scissors. The deepest instrument well, 322, holds ten surgical clamps 348.

The instrument wells could be made deeper to accommodate additional surgical instruments, but when the normal number of instruments is used with the deeper well, a spacer could be installed below the instruments so that the top-most instrument is in a position similar to that of the top-most instrument in Fig. 14.

A locking bar or plate extends over the top-most instrument in each instrument well. The locking plates for all wells are similar; only locking plate 390 (Figs. 15-18) is discussed. Instrument well 328 holds five surgical clamps 348 (Fig. 14). Locking plate 390 mounts on shaft 392, which extends into shoulder 366. The locking plate's top face 400 is in the same plane or close to the same plane as the tray's top surface 302a. The locking plate is in its locked position on the upper-most clamp of the five clamps in Fig. 15. The locking plates can be semicircular. By having the curved or semicircular surfaces of all locking plates in their locked orientation, one can look at the tray and determine if all locking plates are in their locked position. See Fig. 15 in which the curved side of each locking bar faces to the right. Having more than one locking bar for larger instruments such as medium and large needle holders 354 and 352 (Fig. 13) may be desirable. Thus, two locking plates 391 lock those instruments. The circular surfaces also face each other. In addition to the viewing advantage provided by semicircular locking plates, the circular outer surface of the locking plates slides more easily over the top-most surgical instrument when the plates are pivoted to their locked position.

The locking plates could have a stop or stops (not shown) such that when each locking plate reaches the locked or unlocked position, the locking plate stops in that position. A stop also could provide touch feedback whether the locking plate is locked or unlocked. The locking plates also may have indicia such as an arrow or other distinct figure. Similarly, the opposite sides of the lacking plates could have contrasting colors. The indicia or contrasting colors make determining if all locking plates are locked easier.

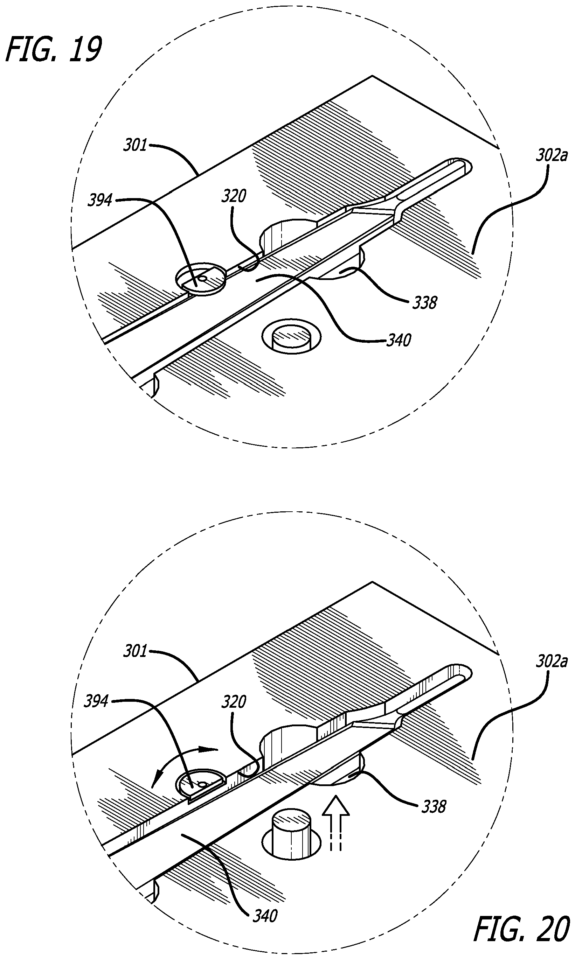

When a person wants to remove an instrument from an instrument well, he or she rotates locking plate 390 from the Fig. 15 position to the Fig. 16 position. See also Figs. 19 and 20, which show locking plate 394 interacting with scalpels 340 in instrument well 320a. When the locking plate is in the Fig. 19 position, it is over a portion of the uppermost scalpel 340 and blocks removal of the instrument from instrument well 320. In the Fig. 20 position, the locking plate pivots out of contact with the scalpel, which allows removal of the scalpels.

When the two halves 301 and 303 of the tray are moving between their open and closed positions, locking plates e.g., plate 394, prevent the surgical instruments from falling out of the instrument wells.

Before surgery begins, the instrument wells are loaded with the correct number of the proper instruments, and all locking plates are rotated to the locked orientation. Therefore, each instrument is secured in its respective instrument well. The surgeon or assistant opens all the locking plates, e.g., plate 390, of fully loaded tray 300. Of course, not all locking plates must be opened in the beginning, but doing so may be more convenient.

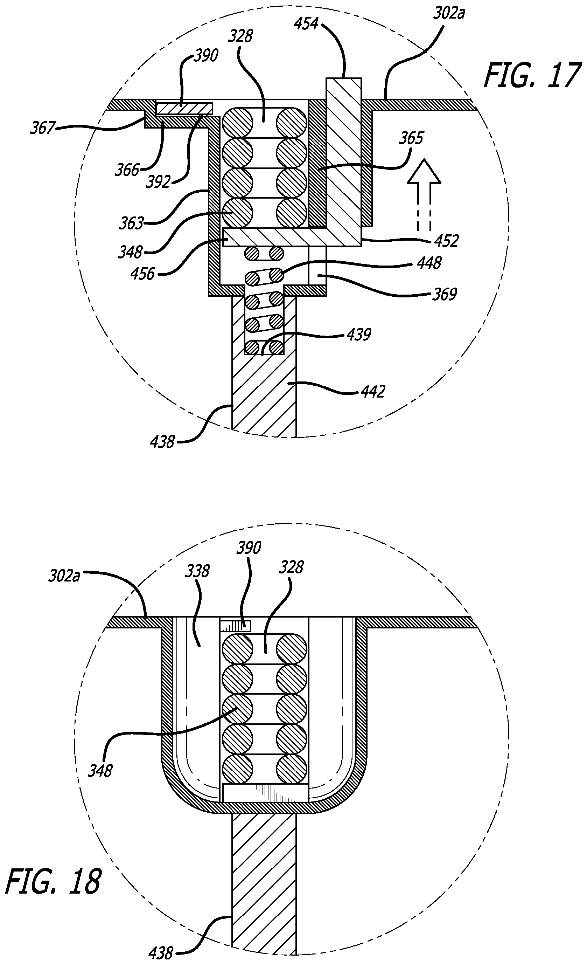

When the locking plate is pivoted to its unlocked orientation, spring 448 raises the instruments 348 and plunger 452. Compare Figs. 15, 16 and 17. As the plunger rises, the top part of upright portion 454 projects to a position flush or close to flush with surface 302a (Fig. 16) to a position about the surface (Fig. 17). Sidewalls 363 and 365 form instrument well 328, and sidewall 365 has a slot 369 that receives arm 456 of plunger 452. The top of the slot limits the distance the plunger can move upward. Thus, as Fig. 17 shows, the top part of the plunger extends above surface 302a.

As the surgery proceeds, the surgeon and his or her staff use the instruments as necessary until the surgery is finished. Then the instruments are returned to their original, respective instrument well. If the same type and size of surgical instrument fits into two or more instrument wells, the instruments can be returned to any of the proper wells. The top of each plunger only retracts from above the top surface 302a when the correct number of instruments is returned to the proper instrument well. Thus, the top of plunger 452 is pushed below top surface 302a when all five surgical clamps 348 are returned to instrument well 328, and the locking plate is returned to its locked position.

If fewer than five surgical clamps 348 are returned to instrument well 328, the top of plunger 452 remains above top surface 302a. If the person refilling tray 300 after surgery sees any plungers extending about top surfaces 302a or 302b he or she knows that at least one instrument was not returned to the tray. However, if all plungers are retracted, the user knows that all instrument wells are refilled. Thus, all instruments in the tray before the surgery have been returned to tray 300, and none is missing
The top of each plunger can be colored to contrast with the color of the top surfaces 302a and 302b to make the visual inspection easier. In addition, the surgeon or staff member can slide a hand over the top surfaces to locate a plunger extending about the top surfaces. If none is felt, the instrument wells are full again and all instruments are back in the tray. If a projecting plunger is felt after all the instruments are reloaded, one knows promptly that an instrument has not been returned. When that occurs, those in the surgical suite can search for the instrument. Because the search can coincide with returning the instruments to the tray, locating all the instruments used occurs at one time and becomes more efficient.

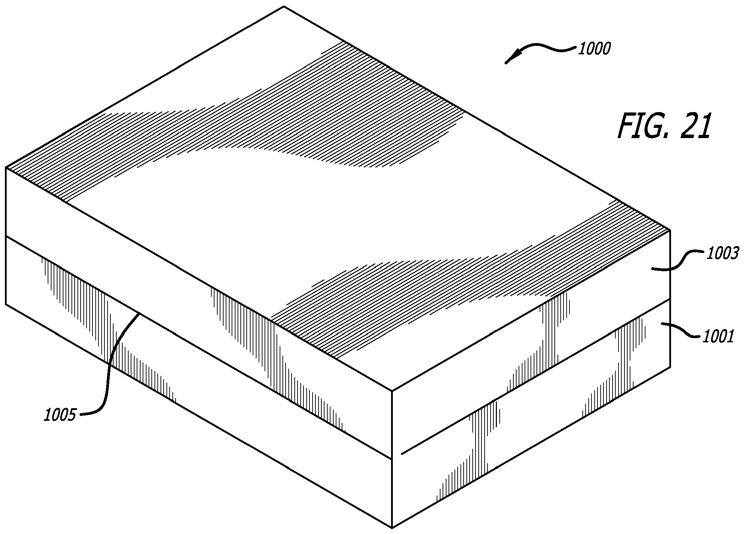

The organizer that Figs. 21, 22 and 23 shows includes tray 1000. The tray's material may be similar to the materials of tray 100 and 300 (Figs. 1 and 10). The material for the tray shown in the drawings is rigid, but the material could be bendable.

Tray 1000 may be divided into at least two sections 1001 and 1003. The two sections may be hinged at 1005 together to allow one section to fold over the other into a closed position. See Fig. 21. The tray may include a lock or other structure for securing the two sections in the closed position.

If the tray is plastic, hinge 1005 could be a plastic living hinge, a type of hinge formed during injection molding. Fabric, metal or plastic or other types of hinges could be used instead of a living hinge.

Tray 1000 may be disposable. However, any non-disposable materials should be able to retain their form when subjected to autoclave temperatures.

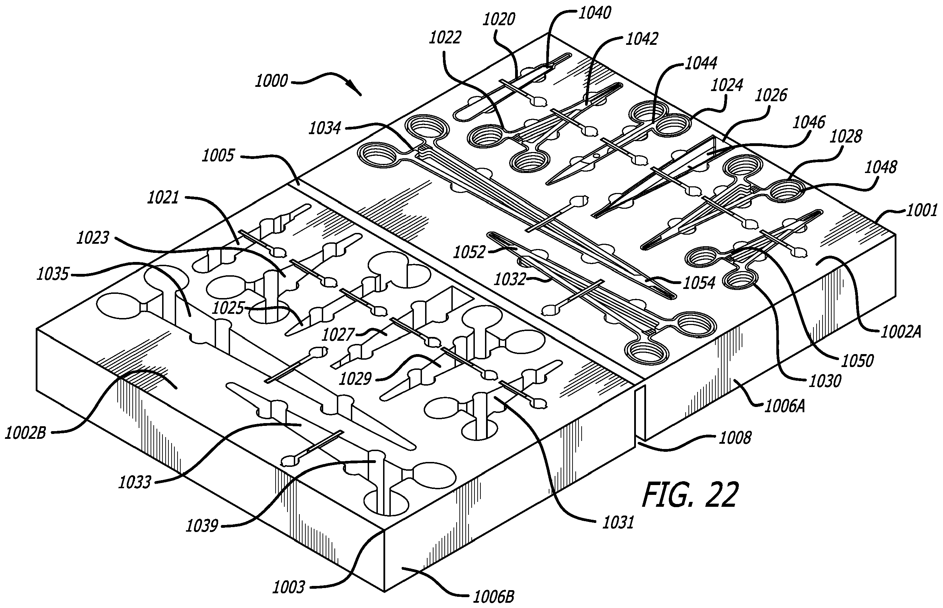

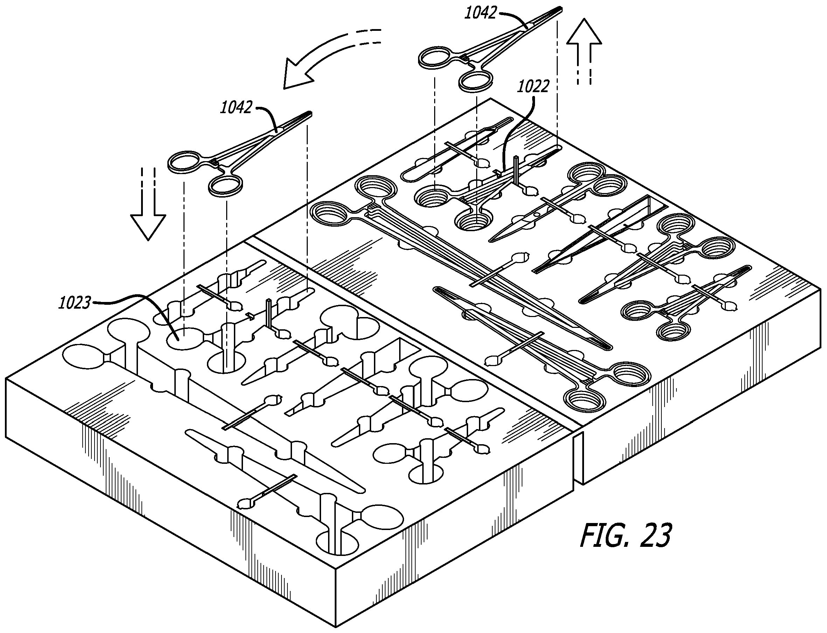

Section 1001 of tray 1000 includes a top surface 1002a. Section 1003 also includes a top surface 1002b. The tray and its sections are rectangular, but other shapes could be acceptable. The tray also has depending sidewalls, only three of which, 1004, 1006a and 1006b, are visible in Figs. 22 and 23. The two sections 1001 and 1003 may be spaced apart along gap 1008 when the tray is open (Figs. 22 and 23).

Indentations in top surfaces 1002a and 1002b of tray 1000 form instrument wells that receive surgical instruments. Section 1001 in Figs 22 and 23 has eight first instrument wells, 1020, 1022, 1024, 1026, 1028, 1030, 1032 and 1034, and section 1003 has eight more second, complimentary instrument wells 1021, 1023, 1025, 1027, 1029, 1031 and 1033. Depending on the requirements for particular surgeries, the tray could have more or fewer instrument wells.

"Complimentary" in the previous paragraph means that each second instrument well in section 1003 has a shape and depth that generally is the same as a corresponding first instrument well in section 1001. See Figs. 22 and 23. Thus, instrument wells 1021 and 1023 generally have the same shape and depth as respective wells 1020 and 1022 for holding scalpels and surgical clamps, respectively.

The instrument wells may have depressions such as depressions 1038 and 1039 at appropriate places to allow easier gripping of an instrument by one's fingers or with an instrument.

The instrument wells may have different depths to accommodate different numbers of surgical instruments and instruments of different dimensions. This application's discussion about Figs. 3 and 14 already described the depths of the instrument wells relative to the thickness of the surgical instrument and the height of a predetermined number of those instruments. Because those descriptions were complete, Figs. 22 and 23 do not show the varied depth of its instrument wells. In addition, Fig. 14 shows an arrangement including springs that urge the surgical instruments toward the top of the tray. The device that Figs. 22 and 23 show may use that arrangement.

A locking bar extends over the top-most instrument in each instrument well. In the Fig. 1 version, the locking bars such as bar 190 mounts for pivoting above or out of the way from the top of each instrument well. In the Fig. 10 version, a locking bar such as plate 390 slides or pivots over at least part of each instrument well. Figs. 22 and 23 use locking bars 190 that Fig. 1 shows. However, it could use those in the Fig. 10 version or equivalents.

When a surgical procedure begins, tray 1000, which contains sterile surgical instruments, is in the surgical suite. The eight instrument wells, 1020, 1022, 1024, 1026, 1028, 1030, 1032 and 1034, in section 1001 are loaded with the predetermined number of surgical instruments for each instrument well. The eight other instrument wells, 1021, 1023, 1025, 1027, 1029, 1031, 1033 and 1035, in section 1003 begin empty. The top surfaces of sections 1001 and 1003 could be different colors or have indicia to differentiate between the section with instrument wells containing unused surgical instruments and the section containing empty instrument wells.

As the procedure begins, the locking bars are pivoted or moved to the position allowing removal of surgical instruments from their respective wells in first section 1001. The instrument that the surgeon calls for is removed from its instrument well and provided to the surgeon. For example, Fig. 23 shows surgical clamp 1042 removed from instrument well 1022, and then after use, the clamp is returned to instrument well 1021. After the surgeon is finished with each particular instrument, the instrument is returned to its appropriate, complimentary instrument well 1021, 1023, 1025, 1027, 1029, 1031, 1033 or 1035 in section 1003. Returning the surgical instrument to the appropriate instrument well may occur immediately after the surgeon finishes using it. Otherwise, the instrument is set aside and later put into its proper instrument well.

The instrument wells in section 1003 are empty when surgery begins (Figs. 22 and 23). However, the instrument well could be in different locations. For example, loaded well 1020 could be next to empty well 1021, and loaded well 1022 could be next to empty well 1023. The empty wells could be turned relative to the loaded well. Alternatively, some of the empty instrument wells could be on one side of section 1001, and remaining, empty instrument could be on the other side of the section. Other arrangements for the instrument wells and the sections are possible.

The various locking bar structures for the instrument wells on section 1003 should lie flat only when the instrument wells have all the proper surgical instruments. In addition, the locking bars may have different colors or indicia on its faces. The proper color or indicia indicate full instrument wells. The device also could use the plunger arrangement such as the one shown in Figs. 15, 16 and 17.

The sides and bottom of the instrument wells such as instrument well 120 (Figs. 1 and 2) enclose the surgical instruments, which leaves only the tops of the wells open. Especially if the organizer is plastic, which is not a good conductor of heat, high-pressure steam in an autoclave may not sterilize the instrument wells and the surgical instruments in the wells sufficiently. Figs. 25 through 29 show variations in the instrument wells, and Fig. 24 shows a variation in organizer that enhance steam reaching the instruments and the wells.

In Figs. 25, 26 and 27, top surface 1102 of tray 1100 has indentations that form instrument wells for receiving surgical instruments. Fig. 25 shows only one such well 1120, which is shaped to receive scalpels. Compare well 120 in Figs. 1 and 2. Instrument well 1120 may have depressions 1138 at appropriate places to allow a finger or tool to enter the well for ease in removing a surgical instrument from the well.

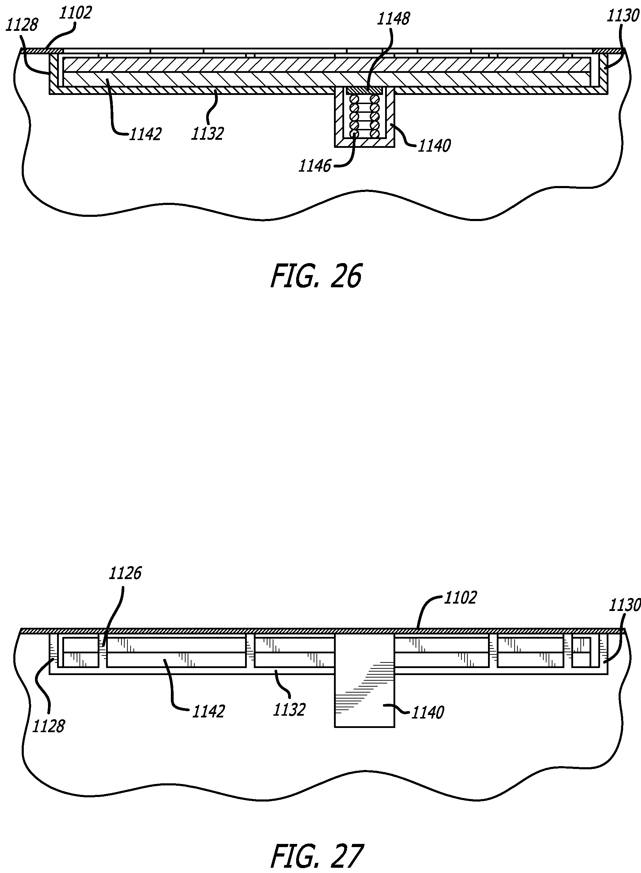

Instead of contiguous bottom and sides, instrument well 1120 is open to allow steam from an autoclave to reach the surgical instruments in the well. See Figs. 25 and 27. Instrument well 1120 is open by using a lattice of supporting members 1118. The supporting member comprises horizontal supports 1122, each of which is flanked by vertical supports 1124 and 1126, extending from the ends of a horizontal support to the top surface 1102.

Figs. 25 and 26 show four supporting members 1118, but the device could have more or fewer. Connector 1132 extends between the supporting members through attachments with horizontal supports 1122. Vertical legs 1128 and 1130 extend upward from connector 1132 at the front and rear of the instrument well. Note that the horizontal supports are different lengths (Fig. 25) to accommodate surgical instruments with different widths from the front to rear of the instrument well. The horizontal supports 1122, vertical supports 1124 and 1126, connector 1132 and vertical legs 1128 and 1130 can be a single integrated member. However, in Fig. 25, housing 1140 for a spring and plunger (described below) divides the integrated member into two parts.

As an alternative, a solid, bottom surface attached to vertical legs could replace the horizontal supports. The solid surface also could have openings to allow steam to enter. This arrangement may provide acceptable sterilization. Likewise, horizontal supports could extend between the bottom of solid sidewalls (with or without openings for steam), such that steam enters the instrument wells through the tops and bottoms of the instrument wells (and any openings in the solid sidewalls.

Using the supporting members such as support 1118 (Fig. 25) leaves substantial space for high-pressure steam to reach the surgical instruments. Other arrangements may be desirable, however. For example, the walls forming the instrument wells could be nominally solid with perforations or other openings through the walls. Screen-like material may accomplish the same function. Likewise, spaced-apart plastic strips could be shaped to form the same shape as the solid walls in Figs. 1 and 2 and other figures. The spacing between the strips allows high-pressure steam to reach the surgical instruments. For strength, short fasteners could connect adjacent strips.

The instrument well in Fig. 25 uses the spring-plunger arrangement similar to the spring and plunger in Figs. 14 through 20. The springs raise the surgical instruments within their respective instrument wells to make them easier to reach. The plunger acts to signal that the respective instrument well contains all its instruments or that some instruments are missing from the well.

Springs such as spring 1146 mount in housing 1140 below instrument well 1120. See Fig. 26. A small plate 1148 attaches to the top of the spring and contacts the bottom-most surgical tool 1142. The spring urges plate 1148 upward, which in turn urges the surgical instruments upward.

Plate 1148 also extends to plunger 1180 (Fig. 25). The plate and plunger may be a single part. Plunger mounts in opening 1181, which conforms to the shape of the plunger (circular in Fig. 25). By urging plate 1148 upward, spring 1146 also urges plunger 1180 upward.

Fig. 25 also shows locking plate 1190 adjacent instrument well 1120. In the Fig. 25 position, the locking plate holds the surgical instruments in place against the upward force from spring 1148. Thus, the locking plate blocks insertion or removal of surgical instruments from the instrument well. The locking plate has a semicircular shape. Pin 1192 mounts the locking plate to spring-plunger housing 1140. The top of tray 1102 has a cutout 1194, which can receive most of the locking bar when it is rotated 180° from its Fig. 25 position. In that rotated position, surgical instruments can be added or removed from the instrument well. The curved or semicircular side of the locking plate allows one to look at all locking plates to determine if all are in their locked position.

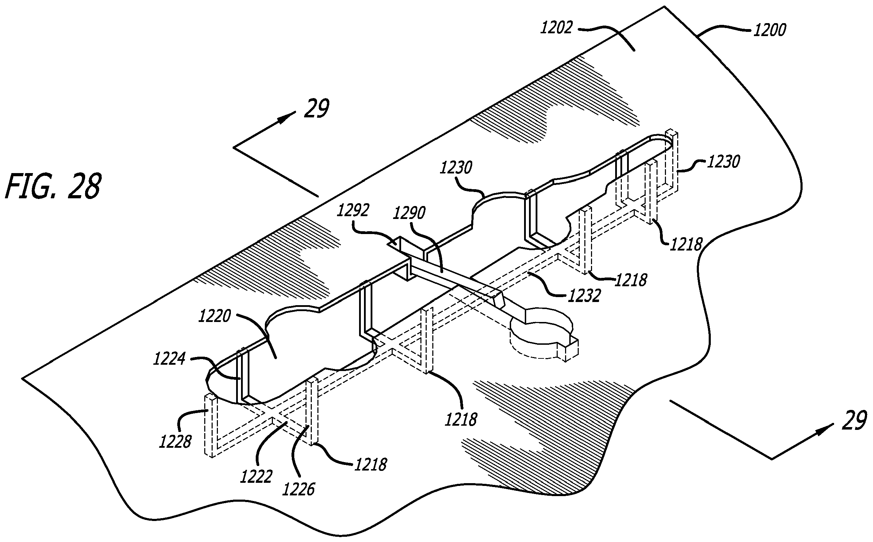

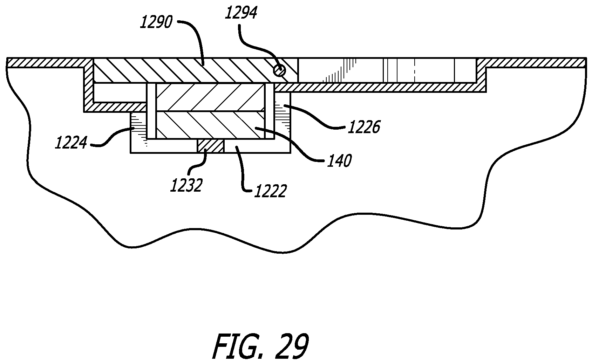

Top surface 1202 of tray 1200 in Figs. 28 and 29 has indentations that form instrument wells for receiving surgical instruments. Fig. 25 shows only one such well 1220, which is shaped to receive scalpels. Instrument well 1220 may have depressions 1238 at appropriate places to allow a finger or tool to enter the well for ease in removing a surgical instrument from the well.

Like the well in Fig. 25, instrument well 1220 also is open to allow steam to reach the surgical instruments in the well as well as the material forming the well. See Fig. 28. Instrument well 1220 has a lattice of supporting members 1218. The supporting member comprises horizontal supports 1222, each of which is flanked by vertical supports 1224 and 1226, extending from the ends of a horizontal support to the top surface 1202.

Instead of using pivoting locking plates 1190 that the Fig. 25 device uses, locking bars 1290 could be used. The locking bars' construction and mounting are similar to those of locking bar 190 in Fig. 1. In particular, the locking bar mounts on pin 1294 in recess 1292. See Figs. 28 and 29. The locking bar can pivot between a position over recess 1220 to prevent adding or removing surgical tools from the recess to a position allowing adding or removing surgical tools.

In addition to the modifications to the instrument well and associated structure in Figs. 24 through 29, tray 1100 (Fig. 24) has open sides 1103, 1104, 1105, 1106 and other sides that are not visible in Fig. 24. The open sides improve sterilization by allowing high-pressure steam to reach the instrument wells and surgical tools more easily.

Though only Fig. 24 through 29 show open sides and open instrument wells, the organizer shown in other figures also could use those features. Even if the organizer uses the same instrument wells for dispensing instruments and returning them after surgery, those organizers could use open sides and open instrument wells.

The description is illustrative, not limiting and is by way of example only. Although this application shows and describes examples, those having ordinary skill in the art will find it apparent that changes, modifications or alterations may be made. Many of the examples involve specific combinations of method, act or system elements, but those acts and elements may be combined in other ways to accomplish the same objectives. Acts, elements and features discussed only in connection with one embodiment are not intended to be excluded from a similar role in other embodiments.

"Plurality" means two or more. A "set" of items may include one or more of such items. The terms "comprising," "including," "carrying," "having," "containing," "involving," and the like in the written description or the claims are open-ended, i.e., each means, "including but not limited to." Only the transitional phrases "consisting of" and "consisting essentially of" are closed or semi-closed transitional phrases with respect to claims. The ordinal terms such as "first," "second," "third," etc., in the claims to modify a claim element do not by themselves connote any priority, precedence, or order of one claim element over another or the temporal order in which acts of a method are performed. Instead, they are used merely as labels to distinguish one claim element having a certain name from another element having a same name (but for use of the ordinal term). Alternatives such as "or" include any combination of the listed items.

## Claims

1. An organizer for holding surgical instruments comprising:
a tray (100) having an upper surface;
a plurality of indentations in the tray forming instrument wells (120, 122, 124, 126, 128, 130, 132, 134) having a shape corresponding to the outer shape of instruments;
each instrument well (120, 122, 124, 126, 128, 130, 132, 134) configured to have a depth corresponding to the height of a stack of a predetermined number of such instruments, at least one instrument well (120, 122, 124, 126, 128, 130, 132, 134) being deeper than at least one other instrument well (120, 122, 124, 126, 128, 130, 132, 134);
**characterized in that** the organizer
further comprising at least a first locking bar (190, 192) at the upper surface of the tray (100) adjacent a first instrument well (1020) and mounted for movement between a first position covering a portion of the first instrument well (1020) to a second position away from the first instrument well (1020), whereby the first locking bar (190, 192) in the first position blocking the removal and addition of a tool out of or into the first instrument well (1020) and whereby the first locking bar (190, 192) in the second position allowing the removal and addition of a tool out of or into the first instrument well (1020),
wherein the first locking bar (190, 192) is pivotably mounted on a pin, the first locking bar (190, 192) having a short section extending from the pin and a longer section opposite the short section, the pin acting as a fulcrum and projecting the short section of the first locking bar (190, 192) above the pin when the first locking bar (190, 192) is returned to first position and its corresponding instrument well (120, 122, 124, 126, 128, 130, 132, 134) contains fewer than the predetermined number of a particular tool.

2. The organizer of claim 1, further comprising at least a second locking bar (190, 192) at the upper surface of the tray (100), adjacent a second instrument well, the second instrument well being spaced apart from the first instrument well (1020), the second locking bar (190, 192) being mounted for movement between a first position covering a portion of the second instrument well to a second position away from the second instrument well, whereby the second locking bar (190, 192) in the first position blocking the removal and addition of a tool out of or into the second instrument well and whereby the second locking bar (190, 192) in the second position allowing the removal and addition of a tool out of or into the second instrument well.

3. The organizer of claim 1 wherein at least a first instrument well (1020) is formed of spaced supports, each spaced support comprising a bottom arm spaced from the top of the instrument well (120, 122, 124, 126, 128, 130, 132, 134), each spaced support further comprising side arms extending from the bottom arm toward the top of the instrument well (120, 122, 124, 126, 128, 130, 132, 134).

4. The organizer of claim 3 further comprising a connector attaching at least two of the spaced supports together.

## Patentansprüche

1. Organisator zum Halten von chirurgischen Instrumenten, umfassend:
ein Tablett (100) mit einer Oberseite;
eine Vielzahl von Einbuchtungen in dem Tablett, die Instrumentenvertiefungen (120, 122, 124, 126, 128, 130, 132, 134) mit einer Form bilden, die der äußeren Form von Instrumenten entspricht;
wobei jede Instrumentenvertiefung (120, 122, 124, 126, 128, 130, 132, 134) konfiguriert ist, um eine Tiefe aufzuweisen, die der Höhe eines Stapels einer vorbestimmten Anzahl solcher Instrumente entspricht, wobei mindestens eine Instrumentenvertiefung (120, 122, 124, 126, 128, 130, 132, 134) tiefer als mindestens eine andere Instrumentenvertiefung (120, 122, 124, 126, 128, 130, 132, 134) ist;
**dadurch gekennzeichnet, dass** der Organisator
weiter mindestens eine erste Verriegelungsstange (190, 192) an der Oberseite des Tabletts (100) umfasst, die benachbart zu einer ersten Instrumentenvertiefung (1020) ist und zur Bewegung zwischen einer ersten Position, einen Abschnitt der ersten Instrumentenvertiefung (1020) abdeckend, und einer zweiten Position weg von der ersten Instrumentenvertiefung (1020) montiert ist, wobei die erste Verriegelungsstange (190, 192) in der ersten Position das Entfernen und Hinzufügen eines Instruments aus oder in die erste Instrumentenvertiefung (1020) blockiert und wobei die erste Verriegelungsstange (190, 192) in der zweiten Position das Entfernen und Hinzufügen eines Instruments aus oder in die erste Instrumentenvertiefung (1020) erlaubt,
wobei die erste Verriegelungsstange (190, 192) schwenkbar an einem Stift montiert ist, wobei die erste Verriegelungsstange (190, 192) einen kurzen Abschnitt, der sich von dem Stift erstreckt, und einen längeren Abschnitt aufweist, der dem kurzen Abschnitt gegenüberliegt, wobei der Stift als Drehpunkt wirkt und den kurzen Abschnitt der ersten Verriegelungsstange (190, 192) über den Stift vorstehen lässt, wenn die erste Verriegelungsstange (190, 192) in die erste Position zurückgeführt wird, und ihre entsprechende Instrumentenvertiefung (120, 122, 124, 126, 128, 130, 132, 134) weniger als die vorbestimmte Anzahl von einem bestimmten Instrument aufweist.

2. Organisator nach Anspruch 1, weiter umfassend mindestens eine zweite Verriegelungsstange (190, 192) an der Oberseite des Tabletts (100) benachbart zu einer zweiten Instrumentenvertiefung, wobei die zweite Instrumentenvertiefung von der ersten Instrumentenvertiefung (1020) beabstandet ist, wobei die zweite Verriegelungsstange (190, 192) zur Bewegung zwischen einer ersten Position, einen Abschnitt der zweiten Instrumentenvertiefung abdeckend, und einer zweiten Position weg von der zweiten Instrumentenvertiefung montiert ist, wobei die zweite Verriegelungsstange (190, 192) in der ersten Position das Entfernen und Hinzufügen eines Instruments aus oder in die zweite Instrumentenvertiefung blockiert und wobei die zweite Verriegelungsstange (190, 192) in der zweiten Position das Entfernen und Hinzufügen eines Instruments aus oder in die zweite Instrumentenvertiefung erlaubt.

3. Organisator nach Anspruch 1, wobei mindestens eine erste Instrumentenvertiefung (1020) aus beabstandeten Trägern gebildet ist, wobei jeder beabstandete Träger einen unteren Arm umfasst, der von der Oberseite der Instrumentenvertiefung (120, 122, 124, 126, 128, 130, 132, 134) beabstandet ist, wobei jeder beabstandete Träger weiter Seitenarme umfasst, die sich von dem unteren Arm zur Oberseite der Instrumentenvertiefung (120, 122, 124, 126, 128, 130, 132, 134) erstrecken.

4. Organisator nach Anspruch 3, weiter umfassend einen Verbinder, der mindestens zwei der beabstandeten Träger miteinander verbindet.

## Revendications

1. Dispositif de rangement pour contenir des instruments chirurgicaux comprenant :
un plateau (100) présentant une surface supérieure ;
une pluralité de creux dans le plateau formant des cavités pour instrument (120, 122, 124, 126, 128, 130, 132, 134) présentant une forme correspondant à la forme extérieure d'instruments ;
chaque cavité pour instrument (120, 122, 124, 126, 128, 130, 132, 134) étant configurée pour présenter une profondeur correspondant à la hauteur d'une pile d'un nombre prédéterminé de tels instruments, au moins une cavité pour instrument (120, 122, 124, 126, 128, 130, 132, 134) étant plus profonde qu'au moins une autre cavité pour instrument (120, 122, 124, 126, 128, 130, 132, 134) ;
**caractérisé en ce que** le dispositif de rangement
comprend en outre au moins une première barre de blocage (190, 192) au niveau de la surface supérieure du plateau (100) de manière adjacente à une première cavité pour instrument (1020) et montée en vue d'un déplacement entre une première position recouvrant une partie de la première cavité pour instrument (1020) et une seconde position éloignée de la première cavité pour instrument (1020), selon lequel la première barre de blocage (190, 192) dans la première position bloque le retrait et l'ajout d'un outil hors de ou dans la première cavité pour instrument (1020) et selon lequel la première barre de blocage (190, 192) dans la seconde position permet le retrait et l'ajout d'un outil hors de ou dans la première cavité pour instrument (1020),
dans lequel la première barre de blocage (190, 192) est montée de manière pivotante sur une tige, la première barre de blocage (190, 192) présentant une section courte s'étendant depuis la tige et une section plus longue opposée à la section courte, la tige servant de pivot et projetant la section courte de la première barre de blocage (190, 192) au-dessus de la tige quand la première barre de blocage (190, 192) est ramenée à la première position et sa cavité pour instrument (120, 122, 124, 126, 128, 130, 132, 134) correspondante contient moins que le nombre prédéterminé d'un outil particulier.

2. Dispositif de rangement selon la revendication 1, comprenant en outre au moins une seconde barre de blocage (190, 192) au niveau de la surface supérieure du plateau (100), de manière adjacente à une seconde cavité pour instrument, la seconde cavité pour instrument étant espacée de la première cavité pour instrument (1020), la seconde barre de blocage (190, 192) étant montée en vue d'un déplacement entre une première position recouvrant une partie de la seconde cavité pour instrument et une seconde position éloignée de la seconde cavité pour instrument, selon lequel la seconde barre de blocage (190, 192) dans la première position bloque le retrait et l'ajout d'un outil hors de ou dans la seconde cavité pour instrument et selon lequel la seconde barre de blocage (190, 192) dans la seconde position permet le retrait et l'ajout d'un outil hors de ou dans la première cavité pour instrument.

3. Dispositif de rangement selon la revendication 1 dans lequel au moins une première cavité pour instrument (1020) est formée de supports espacés, chaque support espacé comprenant un bras inférieur espacé du haut de la cavité pour instrument (120, 122, 124, 126, 128, 130, 132, 134), chaque support espacé comprenant en outre des bras latéraux s'étendant depuis le bras inférieur vers le haut de la cavité pour instrument (120, 122, 124, 126, 128, 130, 132, 134).

4. Dispositif de rangement selon la revendication 3 comprenant en outre un raccord attachant au moins deux des supports espacés ensemble.
